# EUROPEAN PATENT APPLICATION

(11) **EP 3 895 617 A1**
(43) Date of publication of application: **20.10.2021**
(21) Application number: 21167774.5
(22) Date of filing: 12.04.2021
(51) Int. Cl.: A61B 5/276, A61B 5/00, A61B 18/12, A61B 18/16

(54) **RETURN ABLATION PATCH CONTACT SYMMETRY MEASUREMENT**

(30) Priority: 13.04.2020 US 202016846585
(71) Applicant: Biosense Webster (Israel) Ltd, Yokneam, 2066717 (IL)
(72) Inventor: LEVIN, Michael, 2066717 Yokneam (IL); ROTMAN, Eyal, 2066717 Yokneam (IL); ASHKINEZER, Boris, 2066717 Yokneam (IL); BONYAK, Yevgeny, 2066717 Yokneam (IL)
(74) Representative: Carpmaels & Ransford LLP

(57) **Abstract**

A signal return-path symmetry measurement apparatus includes a transformer and monitoring circuitry. The transformer is coupled to first and second return-path electrodes that are attached to a body of a patient and serve as a return path for an electrical signal applied to the patient. The transformer is configured to generate a return-path-difference electrical signal responsive to a difference between electrical signals returning from the first and second return-path electrodes. The monitoring circuitry is configured to generate an electrode symmetry measure responsive to the return-path-difference electrical signal.

## Description

### FIELD OF THE INVENTION

The present invention relates generally to electronic circuits, and particularly to catheter ablation circuitry.

### BACKGROUND OF THE INVENTION

Catheter ablation is a procedure used to ablate a faulty electrical pathway from sections of the hearts of those who are prone to developing cardiac arrhythmias such as atrial fibrillation, atrial flutter, supraventricular tachycardias (SVT) and Wolff-Parkinson-White syndrome (WPW syndrome).

In "Catheter ablation of atrial fibrillation: an update," European Heart Journal (2014) 35, 2454-2459, Haegeli and Calkins describe the current techniques for atrial fibrillation ablation, assess the success rates, discuss new and futures techniques and suggest a guideline for catheter ablation. A summary of the electrical aspects of catheter ablation can be found, for example, in "Catheter Ablation of Cardiac Arrhythmias," by Huang Miller, Fourth Edition, February 2019, pages 8 and 9.

### SUMMARY OF THE INVENTION

An embodiment of the present invention that is described herein provides a signal return-path symmetry measurement apparatus including a transformer and monitoring circuitry. The transformer is coupled to first and second return-path electrodes that are attached to a body of a patient and serve as a return path for an electrical signal applied to the patient. The transformer is configured to generate a return-path-difference electrical signal responsive to a difference between electrical signals returning from the first and second return-path electrodes. The monitoring circuitry is configured to generate an electrode symmetry measure responsive to the return-path-difference electrical signal.

In some embodiments, the electrical signal includes an ablation signal. In an embodiment, the transformer includes first and second primary windings that are respectively coupled to the first and second return-path electrodes, thereby causing the return-path-difference electrical signal, induced in a secondary winding of the transformer, to be responsive to the difference between the electrical signals returning from the first and second return-path electrodes. In an embodiment, the monitoring circuitry is configured to compare the return-path-difference electrical signal to a threshold, and to trigger an alert when the return-path-difference electrical signal exceeds the threshold.

There is additionally provided, in accordance with an embodiment of the present invention, a signal return-path symmetry measurement method, The method includes generating, using a transformer coupled to first and second return-path electrodes that are attached to a body of a patient and serve as a return path for an electrical signal applied to the patient, a return-path-difference electrical signal responsive to a difference between electrical signals returning from the first and second return-path electrodes. An electrode symmetry measure is generated responsive to the return-path-difference electrical signal.

### BRIEF DESCRIPTION OF THE DRAWINGS

The present invention will be more fully understood from the following detailed description of the embodiments thereof, taken together with the drawings in which:
Fig. 1 is a schematic, pictorial illustration of an electro-anatomical system for the administration of intra-cardiac medical ablation procedure, in accordance with an exemplary embodiment of the present invention;
Fig. 2 is a circuit diagram that schematically illustrates an ablation power and monitoring circuit, in accordance with an exemplary embodiment of the present invention; and
Fig. 3 is a flowchart that schematically illustrates a method for measuring symmetry of ablation return electrodes, in accordance with an exemplary embodiment of the present invention.

### DETAILED DESCRIPTION OF EMBODIMENTS

### OVERVIEW

Catheter ablation is an invasive medical procedure whereby a faulty electrical pathway of the heart is ablated, for example, to treat cardiac arrhythmias. A common ablation technique is the administration of a high-power ablation signal to the area of the heart that is to be ablated. Typically, the ablation signal is in the Radio Frequency (RF) range, and the power of the ablation signal is in the order of tens of watts.

The return path of the ablation signal must have a low resistivity, so that the energy of the ablation signal will be mostly absorbed by the ablated tissue. To that end, two return-path electrodes (e.g., two halves of a skin-patch that are electrically isolated from each other) are typically attached to the skin of the patient. A measurement signal (e.g., at a frequency different than the ablation signal frequency) may be applied between the two electrodes in order to assess whether the electrodes are attached properly to the skin; the measurement signal will induce current only if both electrodes are galvanically connected to the skin and, hence, by observing the current that the measurement signal induces, it is possible to tell if any of the connections (or both) is not good. However, this technique does not guarantee that the connections are symmetrical.

Exemplary embodiments of the present invention that are disclosed herein provide improved methods and apparatus for verifying the symmetry of the return-path electrodes that are mounted on the skin-patch. In an exemplary embodiment, the return paths from the two electrodes are wired through two identical primary windings of a transformer, with opposite winding directions, to the return port of the ablation signal. Thus, if the electrical signals through the two return-path electrodes are identical, no magnetic flux will be induced in the transformer. A secondary winding of the transformer is then used to generate an electrical signal proportional to the difference between the currents from the two electrodes, and an indication may be activated if the two signals are sufficiently different from each other.

### SYSTEM DESCRIPTION

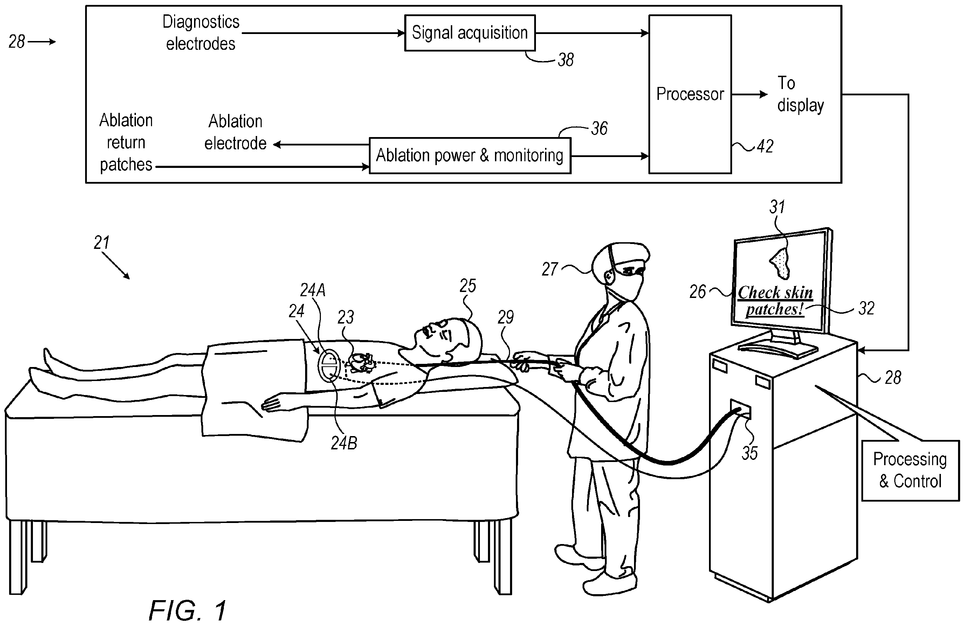

Fig. 1 is a schematic, pictorial illustration of an electro-anatomical system 21 for the administration of intra-cardiac medical procedures, in accordance with an exemplary embodiment of the present invention. In some exemplary embodiments, system 21 is used for administrating a cardiac ablation procedure to a patient.

Fig. 1 depicts a physician 27 using an electro-anatomical catheter 29 to perform an ablation procedure in a heart 23 of a patient 25. The ablation procedure is visualized on a screen 26, which, according to the example embodiment illustrated in Fig. 1, displays an image 31 of the tissue to be ablated. Catheter 29 comprises, at its distal end, one or more diagnostics electrodes, and at least one ablation electrode, which is used to convey an ablation signal to the tissue of the heart that is to be ablated (the diagnostics and ablation electrodes are not shown). The electrodes are coupled, through a connector 35, to Processing and Control circuitry 28. The return path of the ablation signal traverses the blood and other tissues of patient 25, at least one skin-attached patch 24, comprising at least two adjacent electrodes 24A and 24B, and wires that are coupled through connector 35 to the ablation signal ground terminal.

During the ablation procedure, the physician 27 may position the ablation electrode at or near the area of the heart 23 which the physician 27 wishes to ablate, using tracking and guidance techniques which are beyond the scope of the present disclosure. Once the ablation electrode is in place and all other preparatory procedures are completed, the physician 27 may start the ablation by applying a high energy ablation signal through the ablation electrode to the ablation area in the heart. Typically, the ablation signal frequency is in the RF frequency range, and the power is tens of watts (e.g., 480KHz and up to 100W).

As seen in an inset at the top of the figure, Processing and Control circuitry 28 comprises an Ablation-Power-and-Monitoring circuit 36, a Signal-Acquisition circuit 38, and a Processor 42. Signal-Acquisition circuit 38 is configured to measure electrical signals received from the diagnostics electrodes and output the measurement results to Processor 42 (the signal acquisition and the processing of the measurement results by processor 42 are beyond the scope of the present disclosure).

Ablation-Power-and-Monitoring circuit 36, comprising an ablation power generator and monitoring circuitry, is configured to supply the ablation signal to the ablation probe, and to measure the symmetry of the return ablation signals on the return ablation electrodes.

To verify that the return ablation electrodes are connected to the skin, one may inject a measurement signal (at a frequency which is different from the ablation signal frequency) to a first return ablation electrode and test that the signal is received at the second return ablation electrode, thereby verifying that the two electrodes are galvanically connected to the skin of patient 25. Such measurement, however, will not indicate whether the connection of the two electrodes is symmetrical, as the measurement path comprises the two electrodes and two contacts in series.

In the exemplary embodiment illustrated in Fig. 1, the return path of the ablation signal is coupled through return electrodes 24A, 24B and Ablation-Power-and-Monitoring circuit 36 to the return ("ground") terminal of the ablation power source. Ablation-Power-and-Monitoring circuit 36 is configured to measure the difference between the return currents from the two return electrodes 24A, 24B. If one of the skin contacts of skin-patch 24 (that is - electrodes 24A, 24B) is poor, the currents will be different; Ablation-Power-and-Monitoring circuit 36 will then send an indication to processor 42, which may generate an indication 32 to physician 27.

In exemplary embodiments, any suitable indication can be used, including, for example, a flashing warning on screen 26, which may be supplemented by an audible alarm; in yet other exemplary embodiments a symmetry-good indication may be added. The screen 26 may also be utilized to display anatomical feature 31.

Thus, according to the exemplary embodiment illustrated in Fig. 1, the physician 27 may receive an indication that the return paths are good based on the symmetry of the return signals, which may be more accurate than a mere on-off indication.

Fig. 1 mainly shows elements relevant to embodiments of the present invention; in particular, Fig. 1 focuses on the administrating of the ablation power and the measurement of the return path symmetry. Other system elements, such as processor 42, signal-acquisition circuit 38, external ECG recording electrodes and their connections, filtering, digitizing, protecting circuitry, and others are either briefly mentioned or omitted for clarity.

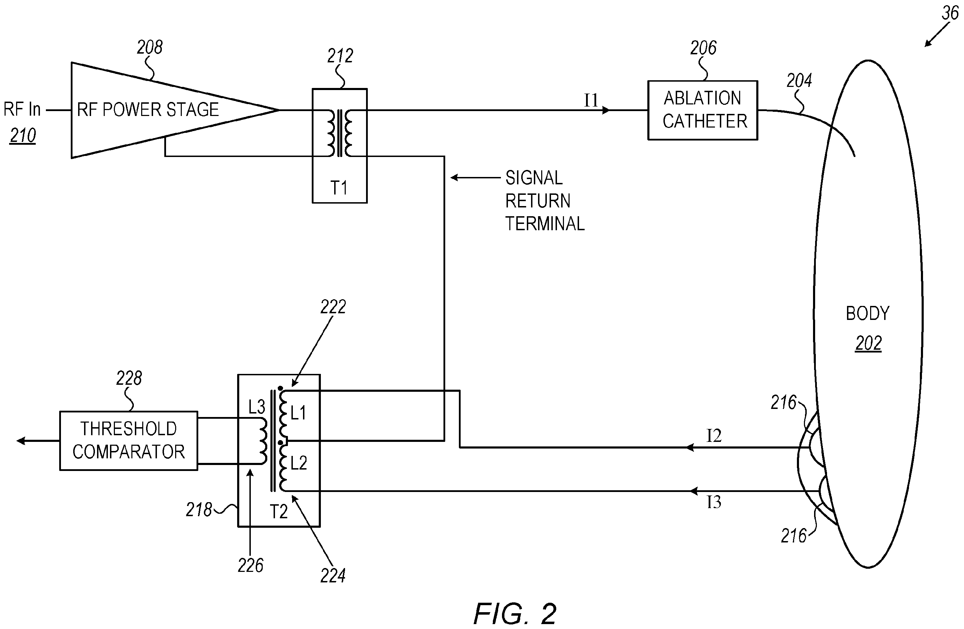

Fig. 2 is a circuit diagram that schematically illustrates ablation power and monitoring circuit 36 (Fig. 1), according to an exemplary embodiment of the present invention. In the present example, a high-power ablation signal is applied to a body 202 of patient 25 (Fig. 1), through an electrode 204 mounted at the distal end of an ablation catheter 206. The ablation power is generated by an RF Power Stage 208 from an RF source 210 (e.g., 480KHz and up to 100W), which is coupled through a transformer T1 212 to the ablation catheter 206. Transformer T1 212 may be used to convert the voltage level of the RF Power stage 208 to a suitable ablation voltage level and/or to increase safety by isolating the ablation current from external power connections. The current administered through ablation catheter 206 to the heart is marked I1.

The return path (to the point marked "signal return terminal") of the ablation signal traverses blood and tissues of patient 25, two return electrodes 216 (that are mounted on a single skin-patch), and a transformer T2 218. The return currents from the two return electrodes 216 are marked I2 and I3. In one exemplary embodiment, catheter 206 corresponds to catheter 29 of Fig. 1, and return electrodes 216 correspond to electrodes 24A and 24B of Fig. 1.

Transformer T2 218 comprises two primary windings L1 222 and L2 224, and a secondary winding L3 226. Windings L1 and L2 are identical in the number of windings and cross section; the directions of windings L1 and L2, however, is opposite, such that the magnetic flux through the transformer will be proportional to the difference between the return currents I2 and I3. Thus, the electrical signal generated in the secondary winding L3 226 will be proportional to the difference 12-13.

The secondary winding L3 226 is coupled to a threshold comparator 228, which is configured to compare the amplitude of the electrical signal on L3 to a preset threshold. If the difference 12-13 is larger than the threshold, threshold comparator 228 sends an indication to processor 42 (Fig. 1), which may, in turn, indicate to the physician 27 that the currents difference exceeds the threshold and, therefore, at least one of the two return electrodes 24 is not properly connected.

In summary, the return currents I2 and I3 would be close to each other if resistances of the patches are good, and different from each other if, for example, one of the resistances is high. The currents are compared by transformer T2 218. Transformer T2 218 is configured to generate an electrical signal proportional to 12-13; threshold comparator 228 is configured to indicate, respective to the generated signal, if one of the electrodes is not properly connected.

As would be appreciated, the example circuit diagram illustrated in Fig. 2 is depicted purely for the sake of conceptual clarity. In alternative exemplary embodiments of the present invention, for example, an additional monitoring circuit is used to check that the sum of 12+13 is greater than a preset threshold (or, alternatively, that I1 is greater than the preset threshold). In an exemplary embodiment, threshold comparator 228 comprises a filter to ignore temporal noisy indication of asymmetry. In some exemplary embodiments, a plurality of pairs of return electrodes 216 are used, mounted on the same or on different skin patches, wherein each pair is wired to two oppositely winded winds of T2, or to a separate T2 transformer.

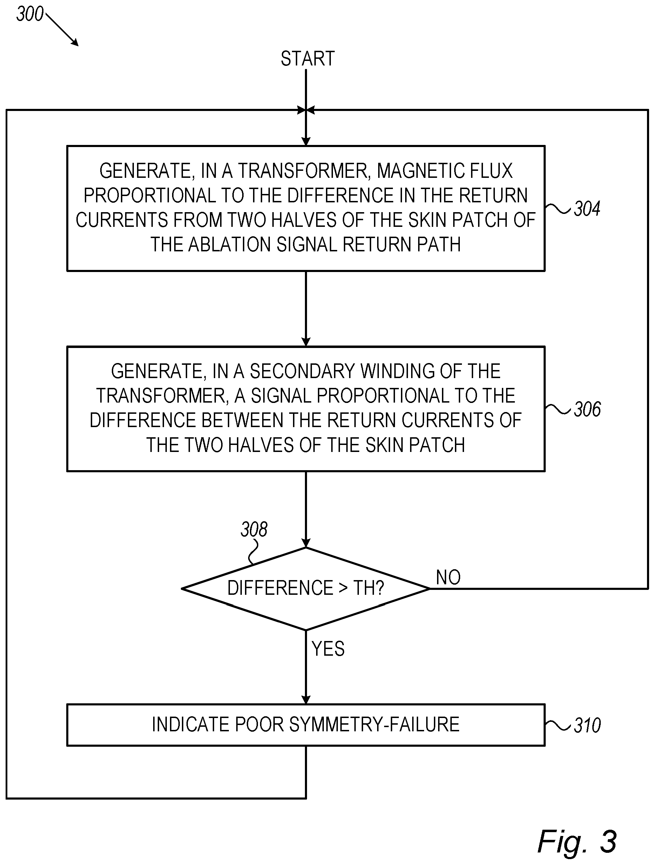

Fig. 3 is a flowchart 300 that schematically illustrates a method for measuring symmetry of ablation return electrodes. The flow is executed by Ablation-Power and Monitoring 36 (Fig. 1).

The flow starts at a Generating-Magnetic-Flux step 304, wherein the Ablation-Power-and-Monitoring circuit generates, in a transformer (e.g., T2 218, Fig. 2) a magnetic flux that is proportional to the difference between return currents of two return electrodes (e.g., 24A, 24B, Fig. 1), which are coupled to oppositely-winded coils of the transformer.

Next, in a Generate-Signal-in-Secondary-Winding step 306, the Ablation-Power-and-Monitoring circuit generates a signal in a secondary winding of the transformer (e.g., L3 226, Fig. 2) that is magnetically coupled to the two oppositely-winded coils. The current is proportional to the sum of the currents in the oppositely winded coils and, therefore, proportional to the difference between the currents through the two return electrodes. Thus, the signal in the secondary winding is proportional to the difference in the currents through the two return electrodes.

Next, in a Compare-Difference-to-Threshold step 308, the Ablation-Power-and-Monitoring circuit compares the signal amplitude to a preset threshold; if the amplitude is greater than the threshold the Ablation-Power-and-Monitoring will enter an Indicate-Poor-Symmetry step 310, and indicate that the return connections are not symmetrical. After step 310, and, in step 308, if the amplitude of the signal is not greater than the threshold, the flowchart restarts from step 304.

Thus, according to the exemplary embodiment illustrated in Fig. 3, a signal proportional to the asymmetry of the two return currents may trigger a poor-contact-quality indication.

As would be appreciated, the exemplary flowchart illustrated in Fig. 3 is depicted purely for the sake of conceptual clarity. In alternative exemplary embodiments, other suitable flows may be used, including, for example, flows wherein a symmetry-good (rather than poor-symmetry) is indicated.

Although the embodiments described herein mainly address catheter ablation return current symmetry, the methods and systems described herein can also be used in other applications, such as other types of ablation and electro-surgical devices.

It will thus be appreciated that the embodiments described above are cited by way of example, and that the present invention is not limited to what has been particularly shown and described hereinabove. Rather, the scope of the present invention includes both combinations and sub-combinations of the various features described hereinabove, as well as variations and modifications thereof which would occur to persons skilled in the art upon reading the foregoing description and which are not disclosed in the prior art.

## Claims

1. A signal return-path symmetry measurement apparatus, comprising:
a transformer, coupled to first and second return-path electrodes that are attached to a body of a patient and serve as a return path for an electrical signal applied to the patient, the transformer configured to generate a return-path-difference electrical signal responsive to a difference between electrical signals returning from the first and second return-path electrodes; and
monitoring circuitry configured to generate an electrode symmetry measure responsive to the return-path-difference electrical signal.

2. The apparatus according to claim 1, wherein the electrical signal comprises an ablation signal.

3. The apparatus according to claim 1, wherein the transformer comprises first and second primary windings that are respectively coupled to the first and second return-path electrodes, thereby causing the return-path-difference electrical signal, induced in a secondary winding of the transformer, to be responsive to the difference between the electrical signals returning from the first and second return-path electrodes.

4. The apparatus according to claim 1, wherein the monitoring circuitry is configured to compare the return-path-difference electrical signal to a threshold, and to trigger an alert when the return-path-difference electrical signal exceeds the threshold.

5. A signal return-path symmetry measurement method, comprising:
generating, using a transformer coupled to first and second return-path electrodes that are attached to a body of a patient and serve as a return path for an electrical signal applied to the patient, a return-path-difference electrical signal responsive to a difference between electrical signals returning from the first and second return-path electrodes; and
generating an electrode symmetry measure responsive to the return-path-difference electrical signal.

6. The method according to claim 5, wherein the electrical signal comprises an ablation signal.

7. The method according to claim 5, wherein the transformer comprises first and second primary windings that are respectively coupled to the first and second return-path electrodes, thereby causing the return-path-difference electrical signal, induced in a secondary winding of the transformer, to be responsive to the difference between the electrical signals returning from the first and second return-path electrodes.
